# EUROPEAN PATENT SPECIFICATION

(11) **EP 1 169 282 B1**
(45) Date of publication and mention of the grant of the patent: **01.06.2005**
(21) Application number: 00921858.7
(22) Date of filing: 07.04.2000
(51) Int. Cl.: C07B 59/00, C07C 229/08, C07C 59/185, C12P 21/00, C07K 1/13

(54) **SITE-SPECIFIC ISOTOPICALLY-LABELED PROTEINS, AMINO ACIDS, AND BIOCHEMICAL PRECURSORS THEREFOR**
SPEZIFISCH MARKIERTE PROTEINE, AMINOSÄURE UND BIOCHEMISCHE VORLÄUFER
PROTEINES ET ACIDES AMINES, ISOTOPIQUEMENT MARQUES ET SPECIFIQUES DE SITES, ET PRECURSEURS BIOCHIMIQUES DESTINES A CES PROTEINES ET ACIDES AMINES

(30) Priority: 09.04.1999 US 289517
(43) Date of publication of application: 09.01.2002
(73) Proprietor: ABBOTT LABORATORIES, Abbott Park, IL 60064-6050 (US)
(72) Inventor: FESIK, Stephen, W., Gurnee, IL 60031 (US); AUGERI, David, J., Emerson, NJ 07630 (US)
(74) Representative: Modiano, Guido, Dr.-Ing.
(86) International application number: PCT/US2000/009296
(87) International publication number: WO 2000/061525

(56) References cited:
- US-A- 5 698 401
- L. K. NICHOLSON: "Dynamics of methyl groups in proteins as studied by proton-detected 13C NMR spectroscopy. Application to the leucine residues in staphylococcal nuclease" BIOCHEMISTRY., vol. 31, no. 23, 1992, pages 5252-5263, XP002146810 AMERICAN CHEMICAL SOCIETY. EASTON, PA., US ISSN: 0006-2960
- W. A. BONNER: "Beta radiolysis of crystalline 14C-labeled amino acids" JOURNAL OF ORGANIC CHEMISTRY, vol. 43, no. 3, 1978, pages 522-524, XP002146811 EASTON US
- "Registry Handbook Number Section, 1982 Supplement" , AMERICAN CHEMICAL SOCIETY XP002146814 page 654RK, column 2, reg. no. 81202-02-0
- "Registry Handbook Number Section, 1988 Supplement" , AMERICAN CHEMICAL SOCIETY XP002146815 page 2456RQ, column 1, reg. no. 115154-39-7
- D. H. G. CROUT: "Pyrrolizidine alkaloids. The bioynthesis of senecic acid" JOURNAL OF THE CHEMICAL SOCIETY, PERKIN TRANSACTIONS 1, 1972, pages 671-680, XP002146812 LETCHWORTH GB
- D. NERI: "Stereospecific nuclear magnetic resonance assignments of the methyl groups of valine and leucine in the DNA-binding domain of the 434 repressor by biosynthetically directed fractional 13C labeling" BIOCHEMISTRY., vol. 28, no. 19, 19 September 1989 (1989-09-19), pages 7510-7516, XP002146813 AMERICAN CHEMICAL SOCIETY. EASTON, PA., US ISSN: 0006-2960

## Description

### Field of the Invention

The present invention relates to site-specific isotopically-labeled organic compounds and processes for their preparation. More particularly, the present invention concerns site-specific isotopically-labeled biochemical precursors of leucine, isoleucine, and valine.

Further the present invention provides methods of preparation of proteins, protein fragments or polypeptides made therefrom.

### Background of the Invention

A recently-developed technique for discovering new drug leads involves the use of nuclear magnetic resonance (NMR) spectroscopy to discover compounds that bind to a particular target molecule such as a protein (*see*, for example, United States Patent Nos. 5,698,401 and 5,804,390, to Fesik, *et al*.). The technique involves the determination of a first two-dimensional ¹⁵N/¹H NMR correlation spectrum of a protein in which nitrogen atom sites have been isotopically enriched with ¹⁵N. This first correlation spectrum is obtained for the protein in the absence of any potential ligand compbund(s). Next a suspected ligand compound, or a mixture of such putative ligand compounds, is mixed with the isotopically enriched protein, and a second NMR correlation spectrum is obtained. The two spectra are compared, and differences between the two spectra provide information about 1) the existence of binding between any ligand and the host protein, 2) the site(s) of binding, and 3) the strength(s) of binding.

The technique described in Fesik, *et al*., *supra,* employs target molecules which have been isotopically enriched with the NMR-detectable ¹⁵N spin nucleus. This method relies upon the genetic modification of a suitable microorganism to express the desired protein, protein fragment, or polypeptide, followed by culturing the modified microorganism in a nutrient medium containing assimilable sources of carbon and nitrogen which include ¹⁵N-labeled nutrients. Comparatively inexpensive commercially available ¹⁵N ammonium salts provided the ¹⁵N source.

However, the application of this NMR drug discovery technique to target molecules isotopically enriched with ¹³C has been hampered by two drawbacks. First, it is comparatively expensive to produce ¹³C-enriched target molecules in any useful quantities.

For example Biochemistry, vol. 31, no. 123, 1992, pages 5252-5263 discloses the synthesis of [5,5'-¹³C₂] leucine via condensation of ethyl acetamido-cyanoacetate and labeled isobutyl bromide and subsequent hydrolysis in the overall yield of the product of 1.6%. The amino acid was biosynthetically incorporated in SNase. Further the production of proteins by genetically modified microorganisms grown in nutrient media containing commercially available uniformly-labeled glucose (glucose-¹³C₆) is expensive. At the time of filing this application, the cost of glucose-¹³C₆ was approximately $480/g.

An example of isotopically enriched protein production is provided by Biochemistry, vol. 28, no. 19, 1989, pages 7510-7516 which discloses a method for biosynthetically producing the proteins of interest wherein the sole source of carbon is ¹³C-labeled and unlabeled glucose.

Alternatively, the production of ¹³C-labeled proteins by including uniformly ¹³C-labeled amino acids in the nutrient medium is similarly expensive. Second, the biomolecules produced using glucose-¹³C₆ or commercially available uniformly ¹³C-enriched amino acids are not ideally suited for the NMR correlation spectra technique. Biomolecules expressed by microorganisms grown in nutrient media containing uniformly ¹³C-enriched starting materials contain adjacent ¹³C-labeled carbon atoms. Since the NMR technique depends upon detection of spatial spin coupling (i.e., the nuclear Overhauser effect), the relatively strong spin-spin coupling of adjacent ¹³C nuclei interferes with the desired observation. There is thus a need for the development of site-specifically ¹³C-enriched amino acids, proteins and polypeptides.

### Summary of the Invention

The instant invention provides biochemical precursors of the site-specific isotopically-enriched amino acids leucine, isoleucine, and valine, as well as the site-specific isotopically-enriched amino acids *per se*. Additionally, proteins, protein fragments and polypeptides containing site-specific isotopically-enriched aminoacyl residues derived from these amino acids, and methods for their production, are also provided. The amino acids and the amino acid biosynthetic precursors are isotopically enriched with either ¹³C or ¹⁴C at the carbon atoms of methyl groups most remote from their carboxyl group. In the labeled amino acids of the present invention, non-adjacent carbon atoms are labeled. In the case where the label is ¹³C, the amino acids of this invention are thus ideally suited for use in the NMR drug discovery technique, since there is no interference with the desired signals by adjacent atom ¹³C-¹³C spin-spin interaction. Moreover, since the amino acids are labeled only at methyl groups, the three magnetically equivalent hydrogen atoms of the methyl group(s) provide strong NMR signals for observation of any effects of coupling with the ¹³C atom(s) to which they are attached.

Specifically, the present invention provides compounds of formula I 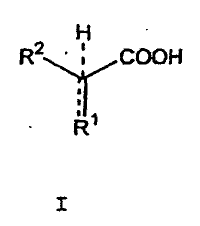 or a salt thereof, wherein R¹ is NH₂, and R² is 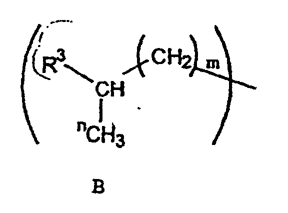 In the formulae presented above, R³ is hydrogen or ⁿCH₃, the dotted line bonds represent valence bonds, m is zero and n, at each occurrence, is 13 or 14.

The present invention provides the site-specific ¹³C- and ¹⁴C-enriched biochemical precursors of leucine, isoleucine, and valine, 2-keto-4-(ⁿC)-butyric acid (formula I above where R¹ is oxygen, R² is B, m is zero, and R³ is hydrogen) and 2-keto-3-(ⁿC-methyl)-4-(ⁿC)-butyric acid (formula I above where R¹ is oxygen, R² is B, m is zero, and R³ is ⁿCH₃). In the foregoing, n represents either 13 (i.e., ¹³C-enriched compounds) or 14 (i.e., ¹⁴C-enriched compounds).

Also provided by the present invention are chemical methods of preparing the site-specific ¹³C- and ¹⁴C-labeled biochemical precursors acids, of formula la as given in claim 1 or salts thereof, which involves reacting a compound of formula IV 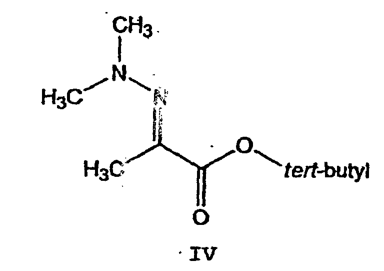 with isotopically-labeled methyl iodide (H₃ⁿCI) to produce a compound of formula V 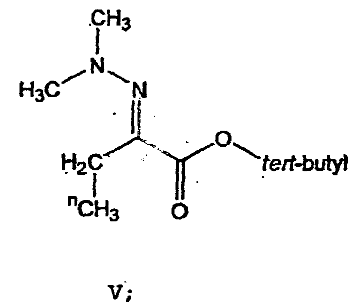 and when R³ is hydrogen removing the protecting *tert*-butyl ester and dimethylhydrazino groups of a compound of formula V to produce 2-keto-4-(ⁿC)-butyric acid; or further when R³ is ⁿCH₃ reacting a compound of formula V with isotopically-labeled methyl iodide (H₃ⁿCI) where n is 13 or 14, to produce a compound of formula VI 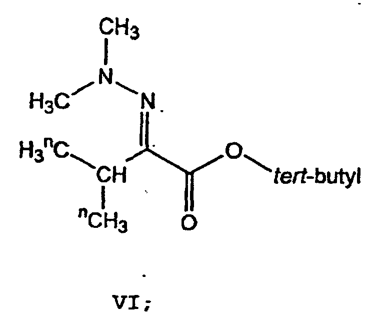 removing the protecting *tert*-butyl ester and dimethylhydrazino groups to produce 2-keto-3-(ⁿC-methyl)-4-(ⁿC)-butyric acid; and optionally salifying the products.

The present invention additionally provides methods for preparing the site-specific ¹³C- and ¹⁴C-labeled amino acids, leucine, isoleucine, and valine. The process involves genetically modifying a microorganism to express a polypeptide containing an amino acid selected from leucine, isoleucine, valine and mixtures thereof; culturing the modified microorganism in a nutrient medium containing assimilable sources of carbon and nitrogen which includes 2-keto-4-(ⁿC)-butyric acid, 2-keto-3-(ⁿC-methyl)-4-(ⁿC) -butyric acid, and salts and mixtures thereof; isolating the resulting expressed polypeptide; and fragmenting the polypeptide and isolating the individual amino acids. The expressed polypeptide is fragmented by conventional methods known in the art including hydrolysis or enzymatic cleavage.

The yield of a particular amino acid may be maximized and the cost minimized by modifying the host microorganism to express a homopolymer of the amino acid, and utilizing the appropriate isotopically enriched biosynthetic precursor in the nutrient medium.

The present invention still further provides a method of preparing a protein, protein fragment, or polypeptide containing amino acyl residues derived from amino acids selected from the group consisting of L-2-amino-3-methyl-5-(¹³C)-pentanoic acid; L-2-amino-3-methyl-5-(¹⁴C)-pentanoic acid ; L-2-amino-4-(¹³C-methyl-5-(¹³C)-pentanoic; L-2-amino-4- (¹⁴C-methyl-5-(¹⁴C)-pentanoic acid; L-2-amino-3-(¹³C-methyl)-5-(¹³C)-butanoic acid; and L-2-amino-3-(¹⁴C-methyl)-5-(¹⁴C)-butanoic acid which involves genetically modifying a microorganism to express a pre-determined protein, protein fragment or polypeptide; culturing the modified microorganism in a nutrient medium containing assimilable sources of carbon and nitrogen which includes 2-keto-4-(ⁿC)-butyric acid, 2-keto-3-(ⁿC-methyl) -4- (ⁿC)-butyric acid, and salts and mixtures thereof; and isolating the resulting expressed polypeptide.

### Detailed Description of the Invention

The natural isotopic abundance of ¹³C is 1.11%, and that of ¹⁴C is negligibly low. Thus the probability that any given carbon atom within an organic molecule is ¹³C is normally about 0.0111, and the probability that any given carbon atom is ¹⁴C is quite low. When target proteins are prepared for use in the adapted NMR "screening" or drug discovery process as described by Fesik, *et al*., *supra,* it is desirable that the ¹³C NMR signal be enhanced by increasing the natural ¹³C content of the target molecule being studied. This is accomplished by either uniformly or selectively enriching the target molecule with ¹³C. As used throughout this specification and the appended claims, the terms "uniform enrichment," "uniformly enriching," "uniformly enriched," uniform labeling" and "uniformly labeled" mean increasing to a value greater than 0.0111, by synthetic means, the probability that a carbon atom randomly selected throughout the target molecule will be ¹³C. The terms "specific enrichment," "site-specific enrichment," "specifically enriching," "specifically enriched," "specifically labeling" and "specifically labeled" mean increasing to a value greater than 0.0111, by synthetic means, the probability that carbon atoms at one or more specific pre-selected site(s) within the target molecule will be ¹³C.

For example, biomolecules expressed by genetically modified microorganisms grown in a nutrient medium containing uniformly ¹³C-enriched glucose will be uniformly ¹³C enriched. A protein expressed by a genetically modified microorganism grown in a nutrient medium containing an amino acid which is ¹³C-enriched only on the methyl side chain would be specifically enriched by ¹³C at the alanyl residues contained within the expressed protein. Similarly, proteins expressed by the method of this invention will be site-specifically enriched by ¹³C or ¹⁴C at the side-chain terminal methyl groups of leucine, isoleucine, and valine.

The method of the present invention also permits the preparation of site-specifically labeled leucine, isoleucine and valine, proteins, protein fragments, or polypeptides made from these labeled amino acids, and the amino acid biosynthetic precursors with labeled with ¹⁴C as well as ¹³C. Such compounds are useful, for example, in studies of protein metabolism where it is desirable to follow the course and fate of protein degradation by radiometric methods.

Further terms used throughout this specification and the appended claims have their usually accepted meanings. The following specific terms have the ascribed meanings:
"DTT" means dithiothreitol.
"HEPES" denotes N-2-hydroxyethylpiperazine-N'-2-ethylsulfonic acid.
"IPTG" means isopropyl-β-D-thiogalactopyranoside.
"PMSF" refers to α-toluenesulfonyl fluoride.
"SCD" refers to the catalytic domain (residues 81-256) of stromelysin.

The preparation of an exemplary site-specific ⁿC-enriched protein fragment target molecule is set forth below. The particular example shown demonstrates the preparation of the so-called "catalytic domain" of human stromelysin ("SCD"), labeled with site-specific ¹³C-enriched leucine, valine, and isoleucine. While shown with ¹³C-labeled amino acid precursors, the method is equally applicable starting with ¹⁴C-labeled amino acid precursors. A preferred means of preparing adequate quantities of specifically ⁿC-enriched polypeptide-containing target molecules involves the transformation of a host cell with an expression vector containing a polynucleotide encoding the desired polypeptide. The protein or polypeptide protein fragment is expressed by culturing the transformed cell line in a medium containing assimilable sources of carbon and nitrogen well known in the art and including the ⁿC-enriched biochemical precursors of this invention. For site-specific labeling of the protein or protein fragment in accordance with the present invention, assimilable sources for ⁿC labeling of a target polypeptide include ⁿC-labeled biosynthetic precursors of amino acids.

For example, it is known that α-keto-butyrate is the biosynthetic precursor of isoleucine, and that α-keto-isovalerate is the biosynthetic precursor of both valine and leucine. Scheme I below shows how the specifically ⁿC-enriched biosynthetic precursors of leucine, isoleucine, and valine can be synthesized. The Scheme employs the comparatively inexpensive ⁿC-enriched methyl iodide, H₃ⁿCI, as the source for isotopic enrichment to produce ⁿC-terminally-labeled α-keto-butyric acid and α-keto-isovaleric acid.

The use of a uniformly ¹³C-enriched nutrient such as glucose-¹³C₆ has been typically used as a convenient means of introducing ¹³C enrichment into a target compound; however, it is very expensive. Furthermore, a vast majority of the carbon sites in uniformly ¹³C-labeled targets will have a covalently bonded neighbor which is also ¹³C-labeled, introducing ¹³C-¹³C coupling which can negatively impact both the signal-to-noise and the relaxation properties of ¹³C-labeled sites in the target biomolectile. Alternatively, the nutrient medium may include commercially available uniformly ¹³C-labeled amino acids. While this technique reduces the "dilution" of the labeling, it too, is a costly alternative and likewise suffers from the drawback of adjacent carbon atom ¹³C-¹³C spin-spin interactions.

However, the method of the present invention for ⁿC-labeling of a polypeptide target molecule comprises growing the genetically modified cell line in a nutrient medium containing ⁿC-labeled biosynthetic precursors of particular amino acids. Not only are certain of the amino acids in the resulting protein, protein fragment or polypeptide isotopically enriched, those amino acids are site-specifically labeled.

Specifically the amino acid precursors of the invention are labeled α-keto-butyric acid and α-keto-isovaleric acid. The biosynthetic products of these precursors are leucine, isoleucine, and valine, in which particular side-chain methyl groups are ⁿC-eariched. Because the methyl groups each have three hydrogen atoms connected to a ⁿC-labeled carbon atom, when n is 13, the corresponding NMR signals are particularly strong and distinctive.

The synthesis for labeled α-keto-butyric acid and α-keto-isovaleric acid involves the C-methylation of the terminal carbon atom in pyruvic acid with ⁿC-enriched methyl iodide. Normally, the alkylation of α-keto acids such as pyruvate is inherently difficult and is accompanied by decomposition of the enolate intermediate with the formation of numerous side products. However, Spencer, *et al*., *Tetrahedron Letters*, 1975, 3889 and Williams, *et al*., *ibid*., 1990, 5881 have shown that alkylation of the corresponding oxime enolate has been carried out, although alkylation with primary electrophiles (for example, methyl iodide) was problematic. D. Enders, *et al*., *Angew. Chem. Int*. *Eng. Ed*., 1992, 618 and D. Enders, *et al*., *Synlett,* 1992, 901 have demonstrated that alkylation of an N,N-dimethylhydrazone of pyruvate is possible, but specifically mentioned that the bulky 2,6-dialkyl phenyl ester was necessary to prevent self acylation. Representative compounds of the present invention include the following:
2-keto-4-(¹³C)-butyric acid or a salt thereof;
2-keto-4-(¹⁴C)-butyric acid or a salt thereof;
2-keto-3-(¹³C-methyl)-4-(¹³C)-butyric acid or a salt thereof;
2-keto-3-(¹⁴C-methyl)-4-(¹⁴C)-butyric acid or a salt thereof;

Although the specific compounds named above have been designated as having ¹³C- or ¹⁴C-isotopes at specific sites in the compound, it will be understood by those of ordinary skill in the art that the carbon atoms at these sites in the compounds will not be completely ¹³C or ¹⁴C labeled. The degree of isotopic substitution or "enrichment" at each molecular site depends upon the corresponding degree of enrichment contained in the starting materials utilized in the synthesis.

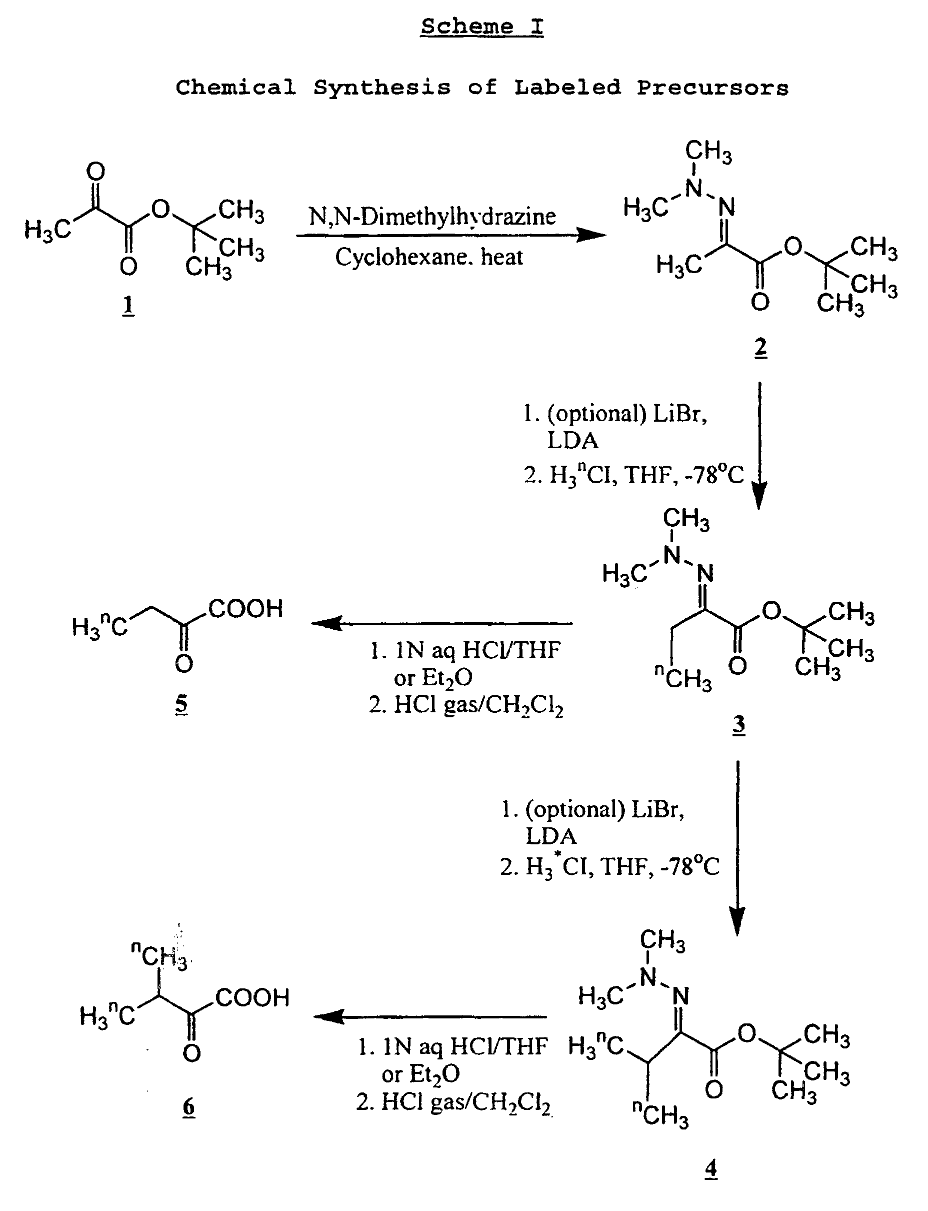

In Scheme I, *tert*-butyl pyruvate, **1**, is converted to the corresponding N, N-dimethylhydrazone, **2**, by reaction with N,N-dimethylhydrazine in diethyl ether at room temperature. The resulting hydrazone, **2**, is cooled in a tetrahydrofuran solution to -78°C, and treated with lithium bromide, followed by lithium diisopropylamide to form the intermediate aza-allyl enolate. The enolate is alkylated with ⁿC-labeled methyl iodide to produce hydrazone **3**. A second course of alkylation of **3** produces the labeled dimethylated hydrazone, **4**. Treatment of **3** and **4** first with aqueous 1N HCl in tetrahydrofuran or diethyl ether (to remove hydrazone) followed by treatment with hydrogen chloride gas in methylene chloride (to remove the t-butyl ester) gives the corresponding ⁿC-terminally labeled α-ketoacids, **5** and **6**. Schemes II, III, and IV illustrate, respectively, how these α-ketoacids are biosynthetically converted into ⁿC-leucine, isoleucine and valine. In all of the Schemes, the site(s) of isotopic enrichment are indicated by asterisks.

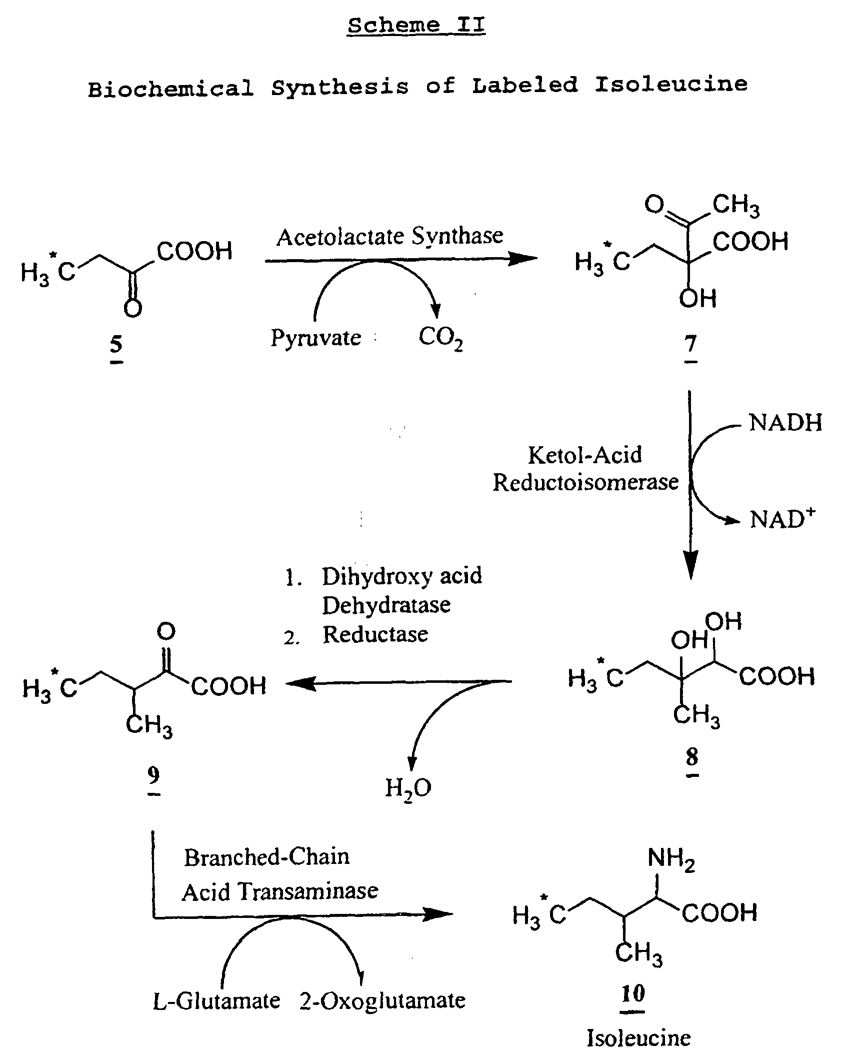

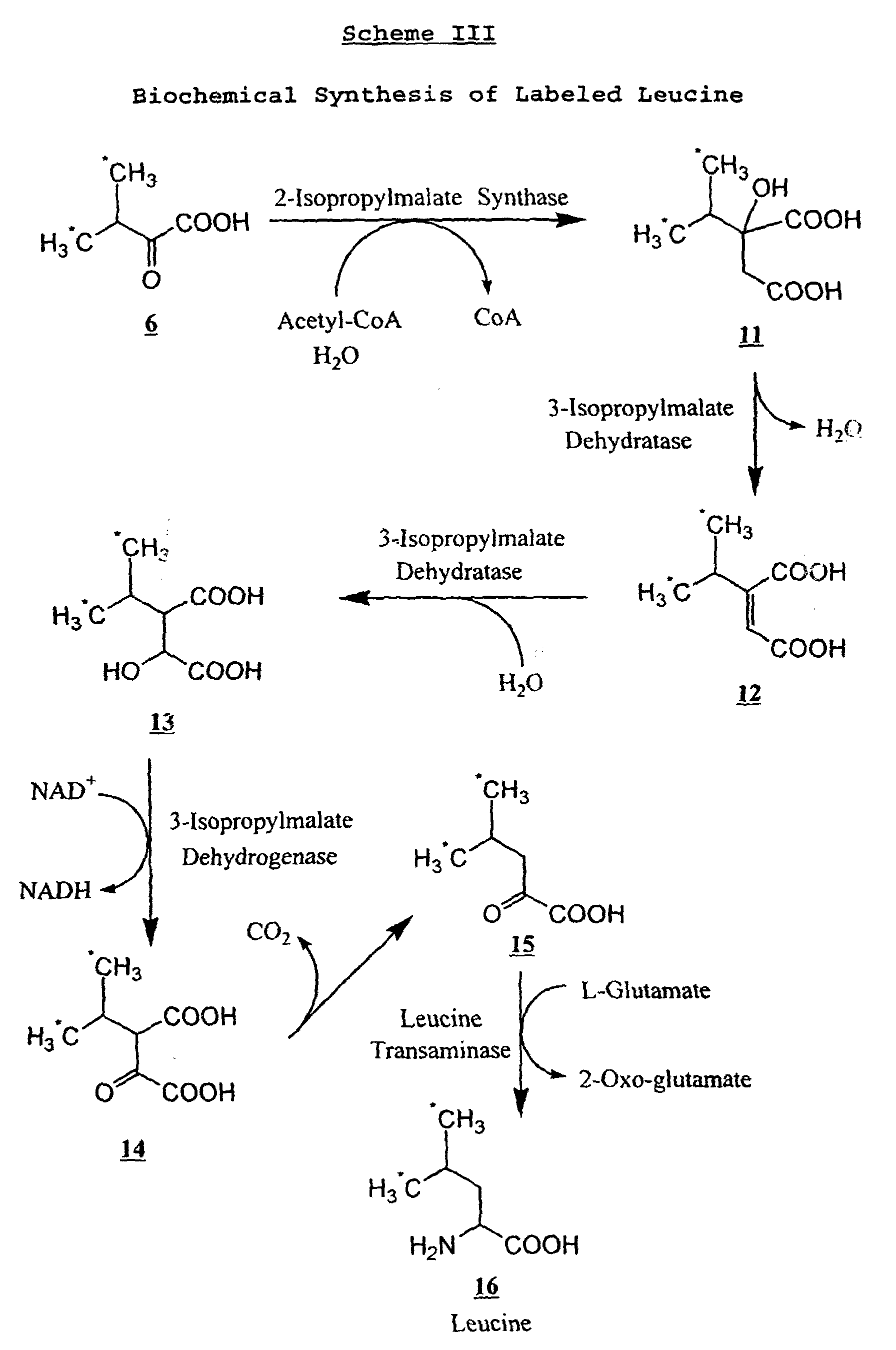

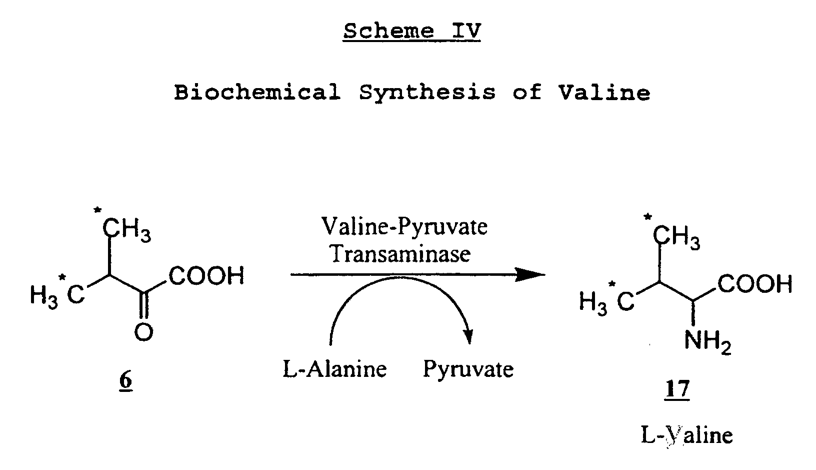

Means for preparing expression vectors that contain polynucleotide sequences coding specific polypeptides and for transforming host cells with those vectors are well known in the art. (See, for example R. W. Old, *et al*., *Techniques of Gene Manipulation,* Blackwell Science, London, 1994, and similar treatises in the field.) Likewise, methods for culturing the transformed cells to express the coded polypeptide and for isolating, purifying and refolding the polypeptide are also well known in the art. Examples presented below describe the production of ¹³C-enriched samples of the 81-256 amino acid catalytic region of human stromelysin (SCD) from modified *E. coli*.

### EXAMPLES

### Example 1

### Preparation of Uniformly ¹³C-Enriched Catalytic Domain of Human Stromelysin (SCD)

The 81-256 fragment (SEQ ID NO: 1) of stromelysin (SCD) is prepared by inserting a plasmid which codes for the production of the protein fragment into an *E. coli* strain and growing the genetically-modified bacterial strain in a suitable culture medium. The protein fragment is isolated from the culture medium, purified, and subsequently used in the two-dimensional NMR analysis of its affinity with test compounds in accordance with the method of this invention. The procedures for the preparation processes are described below.

Human skin fibroblasts (ATCC No. CRL 1507) are grown and induced using the procedure described by Clark, *et al*., *Archiv. Biochem. and Biophys.,* 241: 36 (1985). Total RNA is isolated from 1 g of cells using a RNAgents® Total RNA Isolation System Kit (Promega Corp., 2800 Woods Hollow Road, Madison, WI 53711, USA) following the manufacturer's instructions. A 1 µg portion of the RNA is denatured by heating at 80°C for five minutes and then subjected to reverse transcriptase PCR using a GenAmp® RNA PCR kit (Applied Biosystems/Perkin-Elmer) following the manufacturer's instructions.

Nested PCR is performed using first primers (a) GAAATGAAGAGTCTTCAA (SEQ ID NO: 2) and (b) GCGTCCCAGGTTCTGGAG (SEQ ID No. 3) and thirty-five cycles of 94°C, two minutes; 45°C, two minutes; and 72°C, three minutes. This is followed by re-amplification with internal primers (c) TACCATGGCCTATCCATTGGATGGAGC (SEQ ID NO: 4) and (d) ATAGGATCCTTAGGTCTCAGGGGA GTCAGG (SEQ ID NO: 5) using thirty cycles under the same conditions described immediately above to generate a DNA sequence coding for amino acid residues 1-256 of human stromelysin.

The PCR fragment is then cloned into PCR cloning vector pT7BIue® (Novagen, Inc.) according to the manufacturer's instructions. The resulting plasmid is cut with NcoI and BamHI and the stromelysin fragment is sub-cloned into the expression vector pET3d (Novagen, Inc.), again using the manufacturer's instructions.

A mature stromelysin expression construct coding for amino acid residues 81-256 plus an initiating methionine aminoacyl residue is generated from the 1-256 expression construct by PCR amplification. The resulting PCR fragment is first cloned into the pT7BIue® vector (Novagen, Inc.) and then sub-cloned into the pET3d vector (Novagen, Inc.), using the manufacturer's instructions in the manner described above, to produce plasmid pETST-83-256. This final plasmid is identical to that described by Qi-Zhuang, *et al.*, *Biochemistry,* 31: 11231 (1992) with the exception that the present plasmid codes for a peptide sequence beginning two amino acids earlier, specifically at position 81, in the sequence of human stromelysin. Plasmid pETST-83-256 is transformed into *E. coli* strain BL21(DE3)/pLysS (Novagen, Inc.) in accordance with the manufacturer's instructions, to generate an expression strain, BL2l(DE3)/pLysS/pETST-255-1.

A pre-culture medium is prepared by dissolving 1.698 g of NaH₂PO₄•7H₂O, 0.45 g of KH₂PO₄, 0.075 g NaCl, 0.150 g NH₄Cl, 0.3 g U-¹³C-glucose, 300 µl of 1M aqueous MgSO₄ solution, and 15 ml of aqueous CaCl₂ solution in 150 ml of deionized water. The resulting solution of pre-culture medium is sterilized and transferred to a sterile 500 ml baffle flask. Immediately prior to inoculation of the pre-culture medium with the bacterial strain, 150 ml of a solution containing 34 mg/ml, of chloramphenicol in 100% ethanol and 1.5 ml of a solution containing 20 mg/ml of ampicillin is added to the flask contents. The flask contents are then inoculated with 1 ml of glycerol stock of genetically modified *E. coli* strain BL21(DE3)/pLysS/pETST-255- 1. The flask contents are shaken (225 rpm) at 37°C until an optical density of 0.65 is observed.

A fermentation nutrient medium is prepared by dissolving 113.28 g of Na₂HPO₄•7H₂O, 30 g of KH₂PO₄, 5 g NaCl and 10 ml of 1% DF-60 antifoam agent in 9604 ml of deionized water. This solution is placed in a New Brunswick Scientific Micros Fermenter (Edison, NJ) and sterilized at 121°C for 40 minutes. Immediately prior to inoculation of the fermentation medium, the following pre-sterilized components are added to the fermentation vessel contents: 100 ml of a 10% aqueous solution of NH₄Cl, 15 g of uniformly ¹³C-enriched glucose, 20 ml of an aqueous 1M solution of MgSO₄, 1 ml of an aqueous 1M CaCl₂ solution, 5 ml of an aqueous solution of thiamin hydrochloride (10 mg/ml), 10 ml of a solution containing 34 mg/ml of chloramphenicol in 100% ethanol, and 1.9 g of ampicillin dissolved in the chloramphenicol solution. The pH of the resulting solution is adjusted to pH 7.00 by the addition of an aqueous solution of 4N H₂SO₄.

The pre-culture of *E. coli* strain BL21(DE3)/pLysS/pETST-255-1 from the shake flask scale procedure described above is added to the fermenter contents, and cell growth is allowed to proceed until an optical density of 0.48 is achieved. During this process, the fermenter contents are automatically maintained at pH 7.0 by the addition of 4N H₂SO₄ or 4N KOH as needed. The dissolved oxygen content of the fermenter contents is maintained above 55% air saturation through a cascaded loop which increased agitation speed when the dissolved oxygen content dropped below 55%. Air is fed to the fermenter contents at 7 standard liters per minute (SLPM) and the culture temperature is maintained at 37°C throughout the process.

The cells are harvested by centrifugation at 17,000 x g for 10 minutes at 4°C and the resulting cell pellets are collected and stored at -85°C. The wet cell yield is 3.5 g/L. Analysis of the soluble and insoluble fractions of cell lysates by sodium dodecyl sulfate polyacrylamide gel electrophoresis (SDS-PAGE) reveals that approximately 50% of the stromelysin was found in the soluble phase.

The stromelysin fragment prepared as described above is purified employing a modification of the technique described by Ye, *et al*., *Biochemistry,* 31: 11231 (1992). The harvested cells are suspended in 20 mM Tris-HCl buffer (pH 8.0), sodium azide solution containing 1mM MgCl₂, 0.5 mM ZnCl₂, 25 units/ml of Benzonase® enzyme (Benzon Pharma A/S Roskilde, Denmark), and an inhibitor mixture made up of 4-(2-aminoethyl)benzenesulfonyl fluoride ("AEBSF") Leupeptin®, Aprotinin® and Pepstatin® (all at concentrations of 1 mg/ml. AEBSF, Leupeptin®, Aprotinin®, and Pepstatin® are available from American International Chemical). The resulting mixture is gently stirred for one hour and then cooled to 4°C. The cells are then sonically disrupted using a 50% duty cycle. The resulting lysate is centrifuged at 14,000 rpm for 30 minutes and the pellet of insoluble fraction frozen at -80°C for subsequent processing.

Solid ammonium sulfate is added to the supernatant to the point of 20% of saturation and the resulting solution loaded onto a 700 ml phenyl Sepharose fast flow ("Q-Sepharose FF) column (Pharmacia Biotech.). Prior to loading, the Sepharose column is equilibrated with 50 mM Tris-HCl buffer (pH 7.6 at 4°C), 5 mM CaCl2, and 1M (NH₄)₂SO₄. The loaded column is eluted with a linear gradient of decreasing concentrations of aqueous (NH₄)₂SO₄ (from 1M down to 0M) and increasing concentrations of aqueous CaCl₂ (from 5 mM to 20 mM) in Tris-HCl buffer at pH 7.6. The active fractions of eluate are collected and concentrated in an Amicon stirred cell (Amicon Inc.). The concentrated sample is dialyzed overnight in the starting buffer used with the Q-Sepharose FF column, 50 mM Tris-HCl (pH 8.2 at 4°C) with 10 mM CaCl₂.

The dialyzed sample is then loaded on the Q-Sepharose FF column and eluted with a linear gradient comprising the starting buffer and 200 nM NaCl. The purified soluble fraction of the stromelysin fragment is concentrated and stored at 4°C. The pellet is solubilizcd in 8M guanidine-HCl. The solution is centrifuged for 20 minutes at 20,000 rpm and the supernatant added dropwise to a folding buffer comprising 50 mM Tris-HCl (pH 7.6), 10 mM CaCl₂, 0.5 mM ZnCl₂ and the inhibitor cocktail of AEBSF, Leupeptin(R) Aprotinin(R) and Pepstatin(R) (all at concentrations of 1 µg/ml). The volume of folding buffer is ten times that of the supernatant. The mixture of supernatant and folding buffer are centrifuged at 20,000 rpm for 30 minutes. The supernatant from this centrifugation is stored at 4°C and the pellet subjected twice to the steps described above of solubilization in guanidine-HCl, refolding in buffer, and centrifugation. The final supernatants from each of the three centrifugations are combined and solid ammonium sulfate was added to the point of 20% saturation. The resulting solution thus derived from the insoluble fraction is subjected to purification on phenyl Sepharose and Q-Sepharose as described above for the soluble fraction. The purified soluble and insoluble fractions are combined to produce about 1.8 mg of purified stromelysin 81-256 fragment (SCD) per gram of original cell paste, uniformly enriched with ¹³C.

### Example 2

### Preparation of Specifically ¹³C-Enriched Catalytic Domain of Human Stromelysin (SCD)

SCD is expressed by culturing the BL21(DE3)/pLysS/pETST-255-1 modified *E. coli* strain in a medium comprising 2-keto-4-(¹³C)-butyric acid, or a salt thereof, and 2-keto-3-(¹³C-methyl)-4-(¹³C)-butyric acid, or a salt thereof. The methods used for preparation of the genetically-engineered strain of *E. coli*, and for expressing, isolating, and purifying the protein fragment are as described above, except for the use of U-¹²C-glucose, instead of U-¹³C-glucose.

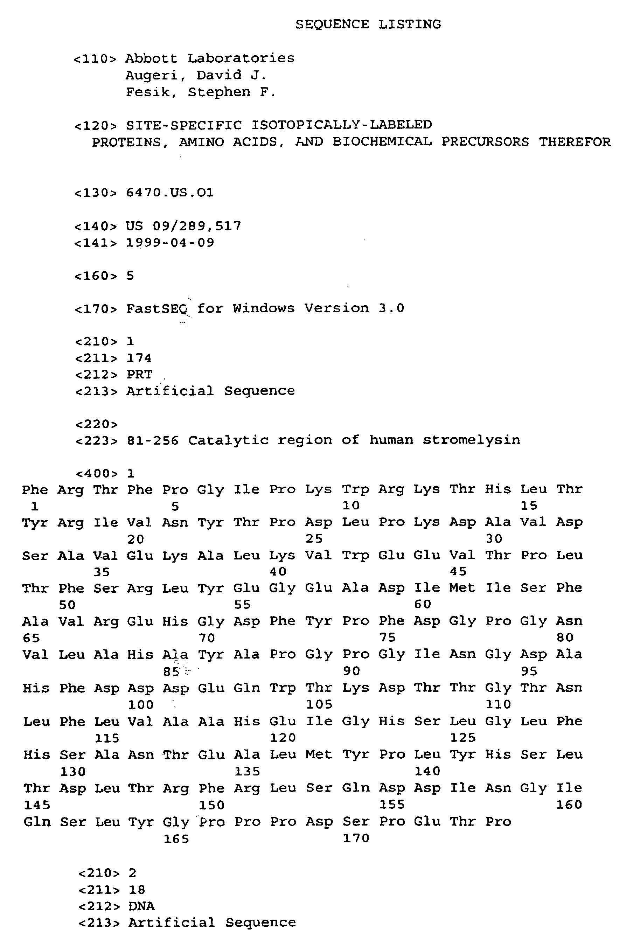

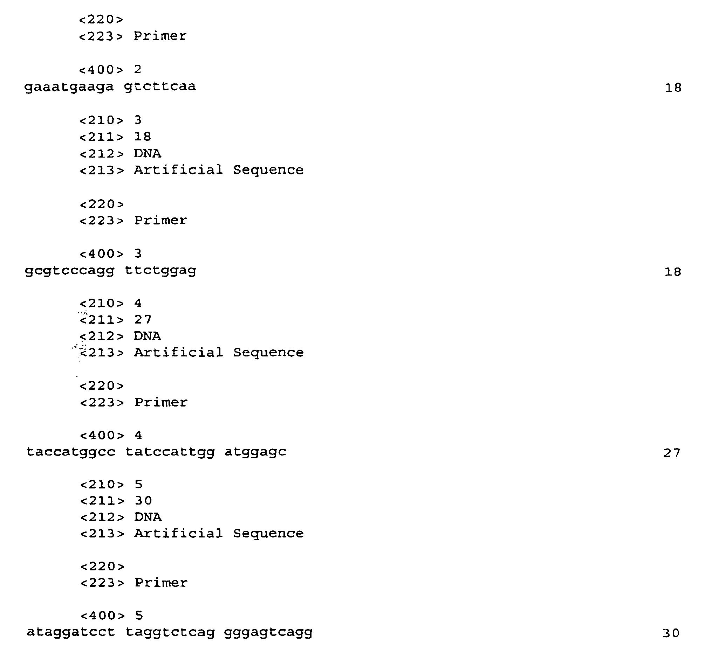

## Claims

1. A compound of formula Ia 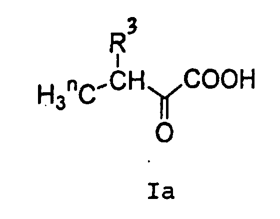 or a salt thereof, wherein R³ is hydrogen or ⁿCH₃ and n, at each occurrence is 13 or 14.

2. A compound according to Claim 1 selected from the group consisting of:
2-keto-4- (¹³C) -butyric acid;
2-keto-3- (¹³C-methyl) -4- (¹³C) -butyric acid;
2-keto-4- (¹⁴C)-butyric acid; and
2-keto-3- (¹⁴C-methyl) -4- (¹⁴C) -butyric acid; or
salts thereof.

3. A method of preparing a compound of formula Ia 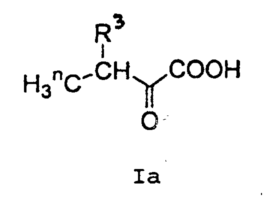 or a salt thereof, wherein R³ is selected from the group consisting of hydrogen and ⁿCH₃, and n at each occurrence is 13 or 14, which comprises
a) reacting a compound of formula IV 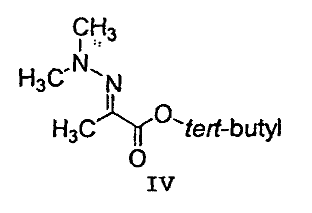 with isotopically-labeled methyl iodide (H₃ⁿCI) to produce a compound of formula V 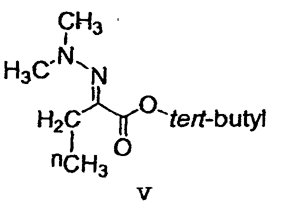 and when R³ is hydrogen
b) removing the tert-butyl ester and dimethylhydrazino groups to produce 2-keto-4 (ⁿC) -butyric acid
or when R³ is ⁿCH₃
c) reacting the product of step a) with isotopically-labeled methyl iodide (H₃ⁿCI), where n is 13 or 14, to produce a compound of formula VI 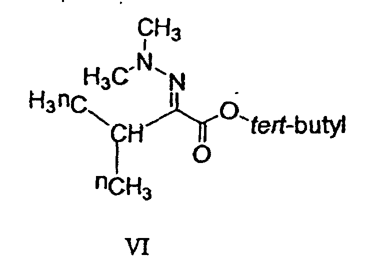 and
d) removing the tert-butyl ester and dimethylhydrazino groups to produce 2-keto-3- (ⁿC-methy 4- (ⁿC) -butyric acid.

4. A method according to Claim 3, which further comprises salifying the reaction product of step b) or step d).

5. A method of preparing a protein, protein fragment or polypeptide containing at least one amino acyl residue selected from the group consisting of 4-(ⁿC-methyl)-5-(ⁿC) leucyl, 5-(ⁿC) -isoleucyl, and 3- (ⁿC-methyl) -4- (ⁿC) -valyl, which comprises
a) genetically modifying a suitable microorganism to express said protein, protein fragment, or polypeptide;
b) culturing said genetically modified microorganism in a nutrient medium containing a compound of formula Ia 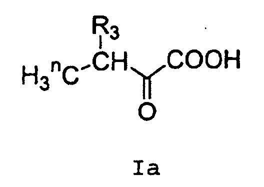 or salt thereof, wherein n, at each occurrence is 13 or 14, and R³ is hydrogen or ⁿCH₃ ; and
c) isolating said protein, protein fragment, or polypeptide

6. The method of claim 5 wherein the protein, protein fragment or polypeptide containing at least one amino acyl residue is selected from 5-ⁿC-isoleucyl and R³ is hydrogen.

7. A method of preparing an amino acid selected from the group consisting of 2-amino-3-(ⁿC-methyl) -4- (ⁿC)- butyric acid, 2-amino-4-(ⁿC-methyl)-5- (ⁿC) - pentanoic acid and 2-amino-3-methyl-5- (ⁿC)-pentanoic acid which comprises
a) genetically modifying a suitable microorganism to express a homopolymer of said amino acid;
b) culturing said genetically modified microorganism in a nutrient medium containing a compound of formula Ia' 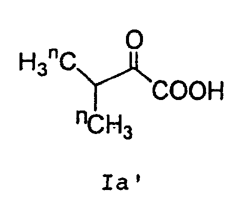 or a salt thereof, when said amino acid is 2-amino-3-(ⁿC-methyl) -4- (ⁿC)- butyric acid and 2-amino-4-(ⁿC-methyl)-5- (ⁿC) - pentanoic acid, where n, at each occurrence, is 13 or 14 or formula Ia'' 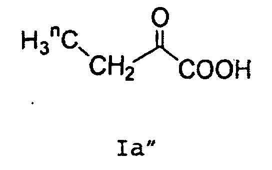 or a salt thereof, when said amino acid is 2-amino-3-methyl-5-(ⁿC)-pentanoic acid;
c) isolating the homopolymer of said amino acid expressed by said genetically modified microorganism; and
d) fragmenting said homopolymer to produce said amino acid.

8. The method of claim 7, wherein said amino acid is 2-amino-3-(ⁿC-methyl) -4- (ⁿC) - butyric acid.

9. The method of claim 7, wherein said amino acid is 2-amino-4-(ⁿC-methyl)-5- (ⁿC) - pentanoic acid.

10. The method of claim 7 wherein said amino acid is 2-amino-3-methyl-5- (ⁿC)-pentanoic acid.

## Patentansprüche

1. Eine Verbindung von Formel Ia 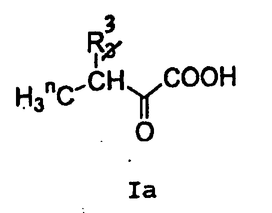 oder ein Salz davon, worin R³ Wasserstoff oder ⁿCH₃ ist und n, jedesmal wenn es vorkommt ist 13 oder 14.

2. Eine Verbindung gemäß Anspruch 1, ist gewählt aus der Gruppe bestehend aus:
2-Keto-4-(¹³C)-buttersäure;
2-Keto-3-(¹³C-Methyl)-4-(¹³C)-buttersäure;
2-Keto-4-(¹⁴C)-buttersäure; und
2-Keto-3-(¹⁴C-Methyl)-4-(¹⁴C)-buttersäure; oder
Salze davon. ,

3. Ein Verfahren zur Herstellung einer Verbindung von Formel Ia 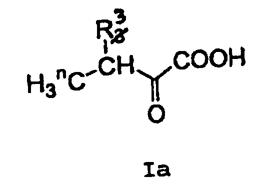 oder ein Salz davon, worin R³ gewählt ist aus der Gruppe bestehend aus Wasserstoff und ⁿCH₃, und n, jedesmal wenn es vorkommt ist 13 oder 14, welches folgendes umfaßt
a) Reagieren einer Verbindung von Formel IV 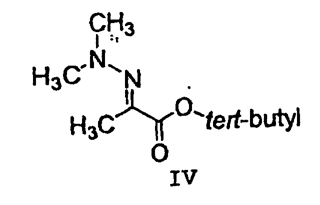 mit isotopisch markiertem Methyljodid (H₃ⁿCI), um eine Verbindung von Formel V herzustellen 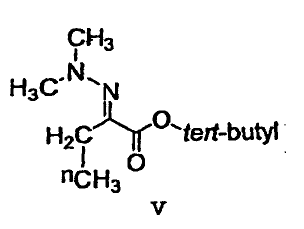 und, wenn R³ Wasserstoff ist,
b) Entfernen des tert-Butylesters und Dimethylhydrazinogruppen, um 2-Keto-4 (ⁿC)-buttersäure herzustellen
oder, wenn R^{3 n}CH₃ ist,
c) Reagieren des Produkts von Schritt a) mit isotopisch markiertem Methyljodid (H₃ⁿCI), worin n 13 oder 14 ist, um eine Verbindung der Formel VI herzustellen 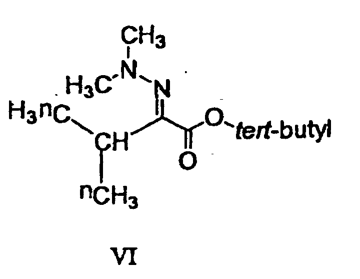 und
d) Entfernen des tert-Butylesters und Dimethylhydrazinogruppen, um 2-Keto-3-(ⁿC-methyl 4-(ⁿC)-buttersäure zu bilden.

4. Ein Verfahren gemäß Anspruch 3, welches weiter die Überführung des Reaktionsprodukts aus Schritt b) oder Schritt d) in ein Salz umfaßt.

5. Ein Verfahren zur Herstellung eines Proteins, Proteinfragments oder Polypeptids, das mindestens einen Aminoacylrest enthält, gewählt aus der Gruppe bestehend aus 4-(ⁿC-Methyl)-5-(ⁿC) leucyl, 5- (ⁿC)-Isoleucyl und 3- (ⁿC-Methyl)-4-(ⁿC)-valyl, welches folgendes umfaßt:
a) genetisches Modifizieren eines geeigneten Mikroorganismus, um das Protein, Proteinfragment oder Polypeptid zu exprimieren;
b) Züchten des genetisch modifizierten Mikroorganismus in einem Nährmedium, das eine Verbindung der Formel Ia 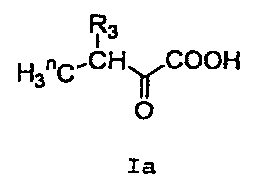 oder ein Salz davon enthält, worin n, jedesmal, wenn es vorkommt, 13 oder 14 ist und R³ Wasserstoff oder ⁿCH₃ ist ; und
c) Isolieren des Proteins, Proteinfragments oder Polypeptids.

6. Das Verfahren von Anspruch 5, worin das Protein, Proteinfragment oder Polypeptid, das mindestens einen Aminoacylrest enthält, gewählt ist aus 5-ⁿC-Isoleucyl, und R³ Wasserstoff ist.

7. Ein Verfahren zur Herstellung einer Aminosäure, gewählt aus der Gruppe bestehend aus 2-Amino-3-(ⁿC-methyl)-4-(ⁿC)-buttersäure, 2-Amino-4-(ⁿC-methyl)-5-(ⁿC)-pentansäure und 2-Amino-3-methyl-5-(ⁿC)-pentansäure, welches folgendes umfaßt
a) genetisches Modifizieren eines geeigneten Mikroorganismus, um ein Homopolymer der Aminosäure zu exprimieren;
b) Kultivieren des genetisch modifizierten Mikroorganismus in einem Nährmedium, das eine Verbindung der Formel Ia' 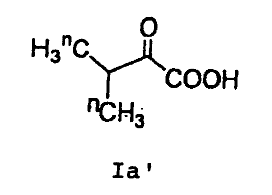 oder ein Salz davon enthält, wenn die Aminosäure 2-Amino-3-(ⁿC-methyl)-4-(ⁿC)-buttersäure und 2-Amino-4-(ⁿC-methyl)-5-(ⁿC)-pentansäure ist, worin n, jedesmal, wenn es vorkommt, 13 oder 14 oder Formel Ia" 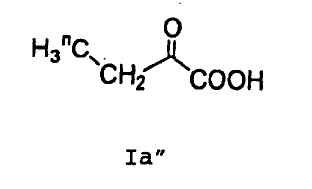 oder ein Salz davon ist, wenn die Aminosäure 2-Amino-3-methyl-5-(ⁿC)-pentansäure ist;
c) Isolieren des Homopolymers der Aminosäure, exprimiert durch den genetisch modifizierten Mikroorganismus; und
d) Fragmentieren des Homopolymers, um die Aminosäure herzustellen.

8. Das Verfahren von Anspruch 7, worin die Aminosäure 2-Amino-3-(ⁿC-methyl)-4- (ⁿC) -buttersäure ist.

9. Das Verfahren von Anspruch 7, worin die Aminosäure 2-Amino-4-(ⁿC-methyl)-5- (ⁿC) -pentansäure ist.

10. Das Verfahren von Anspruch 7, worin die Aminosäure 2-Amino-3-methyl-5-(ⁿC)-pentansäure ist.

## Revendications

1. Composé de formule Ia 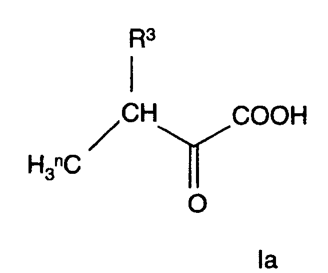 ou sel de celui-ci, où R³ est l'hydrogène ou ⁿch₃ et n, à chaque occurrence, est 13 ou 14.

2. Composé selon la revendication 1, choisi dans le groupe consistant en :
l'acide 2-céto-4-(¹³C)-butyrique ;
l'acide 2-céto-3-(¹³C-méthyl)-4-(¹³C)-butyrique ;
l'acide 2-céto-4-(¹⁴C)-butyrique ; et
l'acide 2-céto-3-(¹⁴C-méthyl)-4-(¹⁴C)-butyrique ; ou leurs sels.

3. Procédé de préparation d'un composé de formule Ia 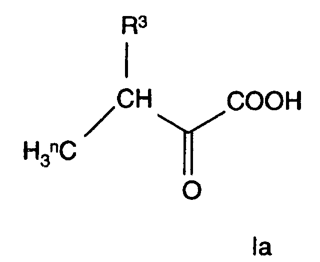 ou d'un sel de celui-ci, où R³ est choisi dans le groupe consistant en l'hydrogène et ⁿCH₃, et n à chaque occurrence est 13 ou 14, qui comprend
a) la réaction d'un composé de formule IV 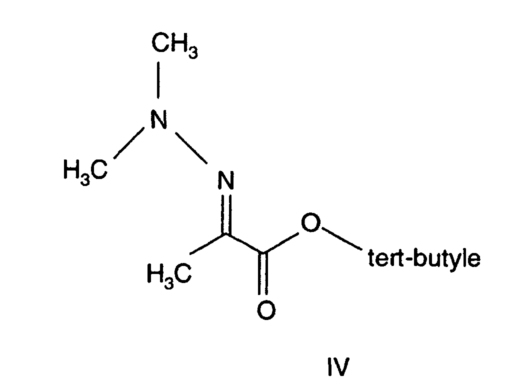 avec l'iodure de méthyle marqué par marquage isotopique (H₃ⁿCI) pour produire un composé de formule V 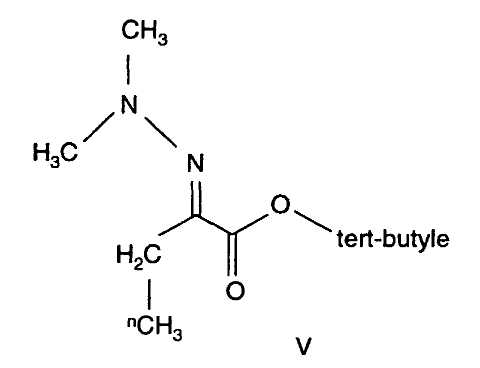 et quand R³ est l'hydrogène
b) le retrait des groupes tert-butylester et diméthylhydrazino pour produire l'acide 2-céto-4 (ⁿC)-butyrique,
où quand R³ est ⁿCH₃
c) la réaction du produit de l'étape a) avec l'iodure de méthyle marqué par marquage isotopique (H₃ⁿCI), où n est 13 ou 14, pour produire un composé de formule VI 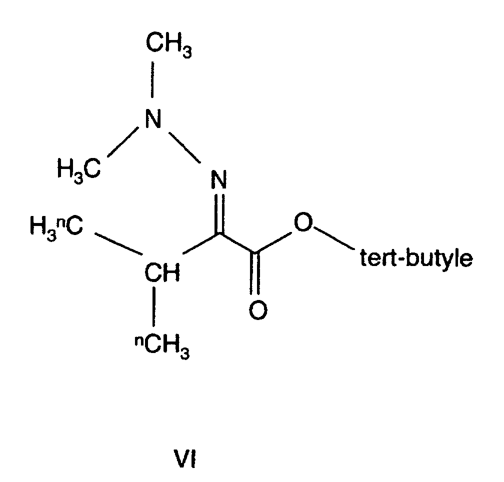 et
d) le retrait des groupes tert-butylester et diméthylhydrazino pour produire l'acide 2-céto-3-(ⁿC-méthyl-4-(ⁿC)-butyrique.

4. Procédé selon la revendication 3 qui comprend en outre la salification du produit réactionnel de l'étape b) ou de l'étape d).

5. Procédé de préparation d'une protéine, d'un fragment de protéine ou d'un polypeptide contenant au moins un résidu aminoacyle choisi dans le groupe consistant en 4-(ⁿC-méthyl)-5-(ⁿC) leucyle, 5-(ⁿC)-isoleucyle et 3-(ⁿC-méthyl)-4-(ⁿC)-valyle, qui comprend
a) la modification génétique d'un microorganisme approprié pour exprimer ladite protéine, ledit fragment de protéine ou ledit polypeptide ;
b) la culture dudit microorganisme modifié génétiquement dans un milieu nutritif contenant un composé de formule Ia 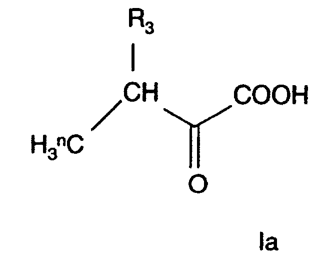 ou un sel de celui-ci, où n, à chaque occurrence, est 13 ou 14, et R³ est l'hydrogène ou ⁿCH₃ ; et
c) l'isolement de ladite protéine, dudit fragment de protéine ou dudit polypeptide.

6. Procédé selon la revendication 5 ou la protéine, le fragment de protéine ou le polypeptide contenant au moins un résidu aminoacyle est choisi parmi 5-ⁿC-isoleucyle et R³ est l'hydrogène.

7. Procédé de préparation d'un aminoacide choisi dans le groupe consistant en l'acide 2-amino-3-(ⁿC-méthyl)-4-(ⁿC)-butyrique, l'acide 2-amino-4-(ⁿC-méthyl)-5-(ⁿC)-pentanoïque et l'acide 2-amino-3-méthyl-5-(ⁿC)-pentanoïque qui comprend
a) la modification génétique d'un microorganisme approprié pour exprimer un homopolymère dudit aminoacide ;
b) la culture dudit microorganisme modifié génétiquement dans un milieu nutritif contenant un composé de formule Ia' 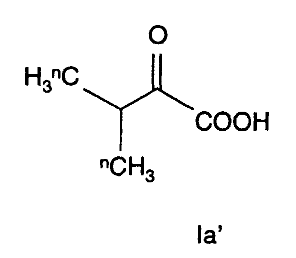 ou un sel de celui-ci, quand ledit aminoacide est l'acide 2-amino-3-(ⁿC-méthyl)-4-(ⁿC)-butyrique et l'acide 2-amino-4-(ⁿC-méthyl)-5-(ⁿC) pentanoïque, où n, à chaque occurrence, est 13 ou 14 ou de formule Ia" 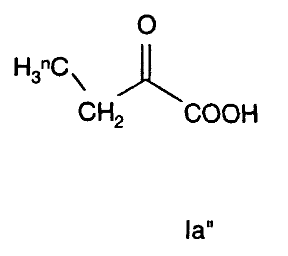 ou un sel de celui-ci, quand ledit aminoacide est l'acide 2-amino-3-méthyl-5-(ⁿC)-pentanoïque ;
c) l'isolement de l'homopolymère dudit aminoacide exprimé par ledit microorganisme modifié génétiquement ; et
d) la fragmentation dudit homopolymère pour produire ledit aminoacide.

8. Procédé selon la revendication 7, où ledit aminoacide est l'acide 2-amino-3-(ⁿC-méthyl)-4-(ⁿC)-butyrique.

9. Procédé selon la revendication 7, où ledit aminoacide est l'acide 2-amino-4-(ⁿC-méthyl)-5-(ⁿC)-pentanoïque.

10. Procédé selon la revendication 7, où ledit aminoacide est l'acide 2-amino-3-méthyl-5-(ⁿC)-pentanoïque.
